(19) Europäisches Patentamt
European Patent Office
Office européen des brevets

(11) Veröffentlichungsnummer: **0 427 993 A2**

# (12) EUROPÄISCHE PATENTANMELDUNG

(21) Anmeldenummer: **90120651.6**

(22) Anmeldetag: **27.10.90**

(51) Int. Cl.5: **C09B 57/00**, C08K 5/3495, C09D 7/12, C07D 471/22

(30) Priorität: **11.11.89 DE 3937633**

(43) Veröffentlichungstag der Anmeldung: **22.05.91 Patentblatt 91/21**

(84) Benannte Vertragsstaaten: **CH DE FR GB LI**

(71) Anmelder: **BAYER AG**
**W-5090 Leverkusen 1 Bayerwerk(DE)**

(72) Erfinder: **Höchstetter, Dr.**
**Färberstrasse 150**
**W-4000 Düsseldorf(DE)**

## (54) Heterocyclische Verbindungen und deren Verwendung als Pigmente und Farbstoffe.

(57) Heterocyclische Verbindungen der Formel

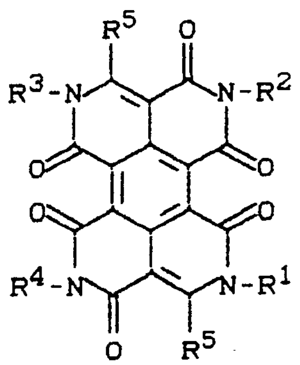

(Ia)

bzw.

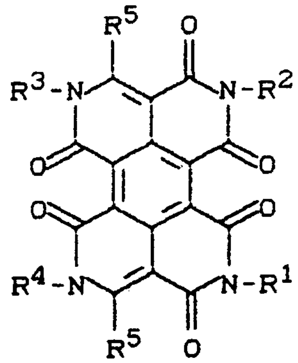

(Ib),

finden Verwendung als Lackpigmente sowie zum Färben und Pigmentieren von hochmolekularem organischen Material.

EP 0 427 993 A2

## HETEROCYCLISCHE VERBINDUNGEN UND DEREN VERWENDUNG ALS PIGMENTE UND FARBSTOFFE

Die Erfindung betrifft heterocyclische Verbindungen der Formel

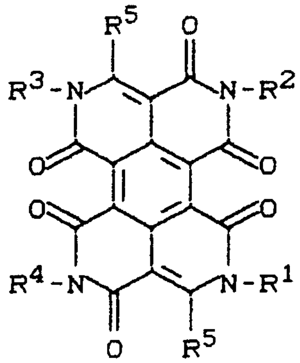

(Ia)

bzw.

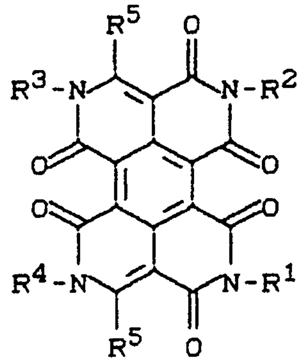

(Ib),

Verfahren zu ihrer Herstellung und ihre Verwendung als Farbstoffe oder Pigmente, insbesondere zum Färben oder Pigmentieren von hochmolekularem organischen Material oder als Lackpigmente, wobei

$R^1$, $R^2$, $R^3$, $R^4$ = H, Alkyl, Cycloalkyl, Aralkyl, Aryl oder Hetaryl,

$R^5$ = Halogen, $NR^1R^2$, $SR^1$, $OR^1$, Aryl, Hetaryl oder OKat, worin

Kat = Kation, wobei für $R^1$ bis $R^4$ = H $R^5$ nicht für OH oder ONa steht.

Die Reste $R^1$, $R^2$, $R^3$ und $R^4$ können gleich oder verschieden sein. Die Alkyl-, Aralkyl-, Cycloalkyl-, Aryl- und Hetarylreste können Substituenten aufweisen. Halogen ist vorzugsweise F, Cl, Br. Alkyl steht beispielsweise für $C_1$-$C_{20}$-Alkyl. Cycloalkyl steht vorzugsweise für mono-, bi- und tricyclisches $C_3$-$C_{10}$-Cycloalkyl, insbesondere für Cyclopentyl und Cyclohexyl. Als Substituenten für die Alkyl- und Cycloalkylreste kommen z.B. in Frage: Halogen wie Cl, Br, F, CN, $OCOR^{10}$, $OR^7$, $COOR^{10}$, $SR^7$, $CONR^8R^9$ und $OCONR^8R^9$,wobei $R^7$ bis $R^{10}$ die unten angegebenen Bedeutungen haben.

Aralkyl ($R^1$ bis $R^4$) steht insbesondere für Phenyl- und Naphthyl-$C_1$-$C_4$-alkyl, wobei die Arylreste, z.B. wie unten für Aryl angegeben, substituiert sein können.

Aryl ($R^1$ bis $R^4$) steht vorzugsweise für solche carbocyclisch-aromatischen Reste, die 1, 2, 3 oder 4, insbesondere 1 oder 2 Ringe enthalten wie Phenyl, Diphenyl und Naphthyl.

Als Hetarylreste ($R^1$ bis $R^4$) stehen vorzugsweise solche heterocyclische (aromatische) Reste, die 1, 2, 3 oder 4, insbesondere 1 oder 2 fünf-, sechs- oder siebengliedrige Ringe enthalten, von denen mindestens einer 1, 2 oder 3, bevorzugt 1 oder 2 Heteroatome aus der Reihe O, N, S enthält. Als heterocyclische Reste seien beispielhaft genannt:

Pyridyl, Pyrimidyl, Pyrazinyl, Triazinyl, Furoyl, Pyrrolyl, Thiophenyl, Chinolyl, Cumarinyl, Benzofuranyl, Benzimidazolyl, Benzoxazolyl, Dibenzofuranyl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naphtharidinyl, Benzimidazolonyl, Benzoxazolonyl, Benzthiazolonyl, Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl,

Chinoxalindionyl, Benzoxazindionyl, Benzoxazinonyl und Naphthalimidyl.

Die Aryl- und Hetarylreste können beispielsweise durch Halogen wie Chlor, Brom und Fluor, -CN, $R^6$, $OR^7$, $SR^7$, $NR^8R^9$, $COOR^{10}$, $COR^{10}$, $NR^8COR^{10}$, $NR^8COOR^{10}$,$NR^8CONR^8R^9$, $NHSO_2R^{10}$, $SO_2R^{10}$, $SO_2OR^{10}$, $CONR^8R^9$, $SO_2NR^8R^9$, $N=N\text{-}R^{11}$, $OCOR^{10}$ und $OCONR^8R^9$ substituiert sein.

$R^6$ bezeichnet gegebenenfalls substituiertes Alkyl, vorzugsweise $C_1$-$C_{18}$-Alkyl, insbesondere $C_1$-$C_4$-Alkyl und gegebenenfalls substituiertes Cycloalkyl, vorzugsweise $C_3$-$C_7$-Cycloalkyl, insbesondere Cyclohexyl und Cyclopentyl.

Als Substituenten der Alkyl- und Cycloalkylreste $R^6$ kommen z.B. in Frage: Halogen wie Cl, Br, F, CN, $OCOR^{10}$, $OR^7$, $COOR^{10}$, $SR^7$, $CONR^8R^9$ und $OCONR^8R^9$.

$R^7$, $R^8$ und $R^9$ bezeichnen Wasserstoff, gegebenenfalls substituiertes Alkyl, insbesondere $C_1$-$C_{18}$-Alkyl, vorzugsweise $C_1$-$C_4$-Alkyl, gegebenenfalls substituiertes Cycloalkyl, insbesondere Cyclohexyl und Cyclopentyl, gegebenenfalls substituiertes Aralkyl, insbesondere Phenyl- und Naphthyl-$C_1$-$C_4$-alkyl, gegebenenfalls substituiertes Aryl, insbesondere Phenyl und Naphthyl und einen gegebenenfalls substituierten heterocyclischen Rest, insbesondere den Rest eines 5- oder 6-gliedrigen heterocyclischen Ringes mit 1, 2 oder 3 Heteroatomen aus der Reihe O, N, S, an den ein Benzolring ankondensiert sein kann.

Die Alkyl- und Cycloalkylreste $R^7$, $R^8$ und $R^9$ können z.B. durch Cl, Br, F, CN, Mono-$C_1$-$C_4$-alkylamino, Di-$C_1$-$C_4$-alkylamino, Phenyl oder Naphthyl, die durch Cl, Br, F, $C_1$-$C_6$-Alkyl und $C_1$-$C_6$-Alkoxy substituiert sein können, oder durch heterocyclische Reste eines 5- oder 6-gliedrigen heterocyclischen Ringsystems mit 1 oder 2 Heteroatomen aus der Reihe O, N, S, an das ein Benzolring ankondensiert sein kann, substituiert sein.

Auch können $R^8$ und $R^9$ zusammen unter Einschluß des N-Atoms einen 5- oder 6-gliedrigen heterocyclischen Ring bilden, z.B. einen Morpholin-, Piperidin- oder Phthalimidring. Die Aryl- und Aralkylreste $R^8$ und $R^9$ können beispielsweise durch Cl, Br, F, $C_1$-$C_{18}$-Alkyl, vorzugsweise $C_1$-$C_4$-Alkyl oder durch $C_1$-$C_{18}$-Alkoxy, vorzugsweise $C_1$-$C_4$-Alkoxy, substituiert sein.

$R^{10}$ bezeichnet Wasserstoff, gegebenenfalls substituiertes Alkyl, insbesondere $C_1$-$C_{18}$-Alkyl, vorzugsweise $C_1$-$C_4$-Alkyl, gegebenenfalls substituiertes Cycloalkyl, insbesondere Cyclopentyl und Cyclohexyl, gegebenenfalls substituiertes Aralkyl, insbesondere Phenyl- und Naphthyl-$C_1$-$C_4$-alkyl, vorzugsweise Benzyl, gegebenenfalls substituiertes Aryl, insbesondere Phenyl und Naphthyl.

Die für $R^{10}$ genannten Reste können wie die entsprechenden Reste $R^8$ und $R^9$ substituiert sein.

$R^{11}$ bezeichnet den Rest einer Kupplungskomponente, vorzugsweise einer Kupplungskomponente aus der Benzol-, Naphthalin-, Acetessigesterarylid-, Pyrazol- oder Pyridonreihe oder einen gegebenenfalls durch Cl, Br, F, $C_1$-$C_{18}$-Alkyl, vorzugsweise $C_1$-$C_4$-Alkyl und $C_1$-$C_{18}$-Alkoxy, vorzugsweise $C_1$-$C_4$-Alkoxy, substituierten Phenylrest.

Kat steht beispielsweise für ein Alkali- oder Erdalkalimetallkation oder Übergangsmetallkation wie Kupfer- und Nickelkationen sowie für $NHR_7R_8R_9$, wobei $R^7$-$R^9$ die oben angegebene Bedeutung besitzen und vorzugsweise für H oder gegebenenfalls substituiertes Alkyl stehen.

Im Rahmen der Formel (Ia/Ib) bevorzugte Verbindungen sind solche der Formel

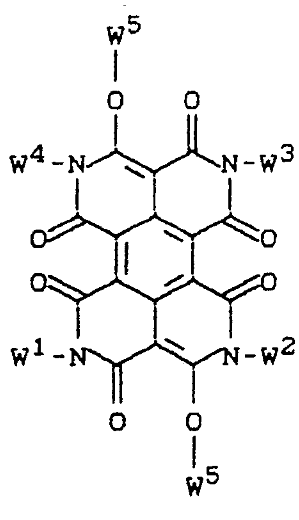

(Ic)

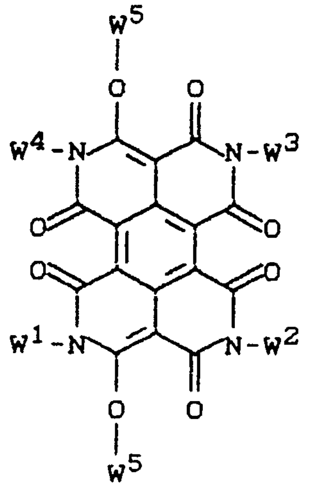

(Id),

worin $W^1$ bis $W^4$ = gegebenenfalls substituiertes Alkyl-bzw. Cycloalkyl und $W^5$ = Wasserstoff oder gegebenenfalls substituiertes Alkyl- oder Cycloalkyl oder Kat. bedeuten.

Besonders bevorzugt sind solohe Verbindungen der Formel (Ic/Id), in denen $W^1$ bis $W^4$ = unsubstituiertes $C_1$-$C_{20}$-Alkyl und $W^5$ = Wasserstoff, gegebenenfalls substituiertes Ammoniumion oder Metallkation bedeuten.

Ganz besonders bevorzugte Verbindungen (Ia/Ib) sind solche der Formel

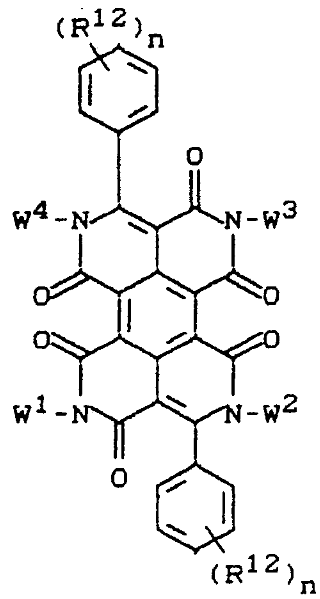

(IIa)

bzw.

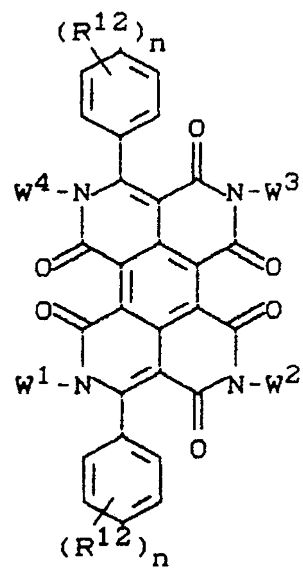

(IIb)

wobei
$W^1$ bis $W^4$ die oben angegebenen Bedeutungen haben, und
$R^{12}$ = Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SOOCOR^{10}$ und $OCONR^8R^9$ mit den obengenannten Substituentenbedeutungen für $R^6$ bis $R^{10}$ und n = 0-3 sein können.

Die in J. Chem. Soc. 1960, S. 3513 ff. beschriebene Oxidation von

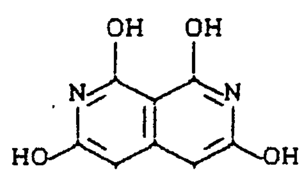

(III)

liefert einen Pentacyclus der vermutlichen Struktur

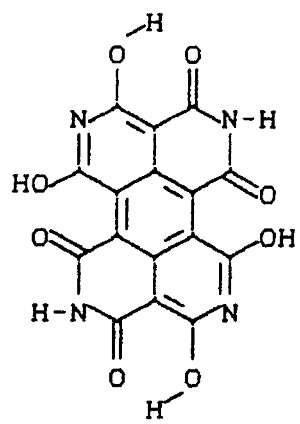

(IVa)

bzw.

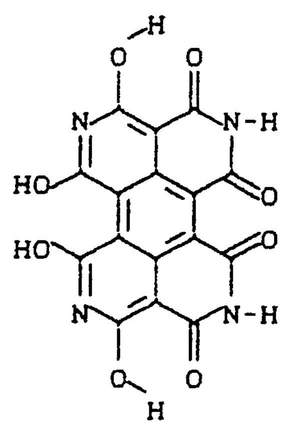

(IVb)

oder eines der möglichen Tautomeren mit der Symmetrie von IVa ($C_{2h}$) bzw. IVb ($C_{2v}$) z.B.

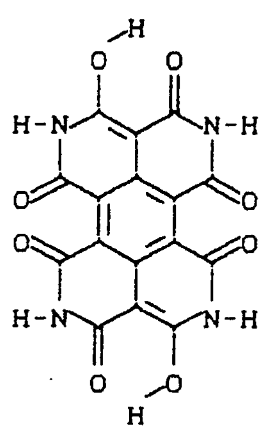

(IVc)

oder deren Salze.

Die Verbindungen Ia bzw. Ib in denen mindestens einer der Reste $R^1$ bis $R^4$ für Alkyl, Cycloalkyl oder Aralkyl steht erhält man ausgehend von den Verbindungen IVa bzw. IVb insbesondere deren Di-Alkalisalzen bzw. Di-Erdalkalisalzen durch Umsetzung mit Alkylierungsmitteln bei etwa 150 bis 250 °C, vorzugsweise 180 bis 220 °C, gegebenenfalls unter Druck und in Gegenwart inerter Lösungsmittel.

Geeignete Alkylierungsmittel sind beispielsweise Alkylhalogenide, Sulfonsäurealkylester oder Schwefelsäurealkylester.

Je nach Menge an Alkylierungsmittel, Temperatur und Reaktionsdauer kann man so Verbindungen (Ia/Ib) mit verschiedenen Alkylierungsgraden erhalten.

So erhält man beispielsweise mit einem großen Überschuß an Methyltosylat eine Tetra-N-methylverbindung der Formel

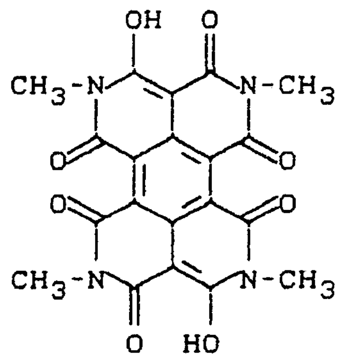

(Va)

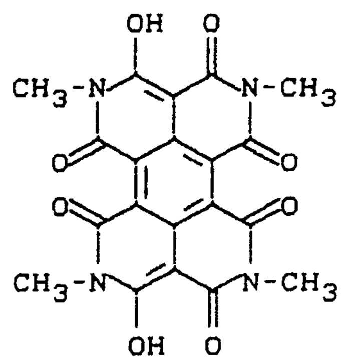

(Vb).

Die Verbindungen Ic bzw. Id mit $W^1$ bis $W^4$ = Alkyl können je nach Kettenlänge der Alkylgruppen als Pigmente für Lacke oder Kunststoffpigmentierung oder als lösliche Farbstoffe zur Kunststoffeinfärbung verwendet werden.

Die Verbindungen der Formel Ic/Id fallen in einer für die Pigmentanwendung geeigneten Form an oder können durch an sich bekannte Nachbehandlungsverfahren in die geeignete Form überführt werden, z.B. durch Losen oder Quellen in starken anorganischen Sauren wie Schwefelsäure und Austragen auf Eis. Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigments können durch Variation der Nachbehandlung beeinflußt werden.

Die Verbindungen der Formel Ic/Id eignen sich aufgrund ihrer Licht- und Migrationsechtheit für die verschiedensten Pigmentapplikationen. So können sie zur Herstellung von sehr echt pigmentierten Systemen in Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch und oxidativ trocknende Lacke, Einbrennlacke, Reaktionslacke, Zweikomponentenlacke, Dispersionsfarben für wetterfeste Überzüge und Leimfarben.

Unter Druckfarben sind solche für den Papier-, Textil-und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylester, Methacrylestern, Acrylnitril, Acrylamid, Butadien, Styrol sowie

Polyurethane und Polycarbonate. Die mit den beanspruchten Produkten pigmentierten Stoffe können auch in beliebiger Form vorliegen.

Verbindungen Ic/Id mit $W^5$ = Kat und $W^1$ bis $W^4$ = Alkyl erhält man aus den entsprechenden Verbindungen mit $W^5$ = H durch Umsetzung mit den entsprechenden Metallhydroxiden, Metallchloriden oder Metallsalzen von Carbonsäuren bzw. den entsprechenden Ammoniumverbindungen bzw. Aminen bei erhöhter Temperatur (20 bis 200 °C) gegebenenfalls in Gegenwart dipolar aprotischer Losungsmittel wie DMF oder DMSO.

Gegebenenfalls setzt man zunächst mit stöchiometrischen Mengen NaOH zum Dinatriumsalz um und unterwirft dieses anschließend einer Metallaustauschreaktion. Die so erhaltenen Ni-, Cu- und Ca-Derivate stellen blaue, sehr lichtechte Pigmente dar.

Verbindungen der Formel

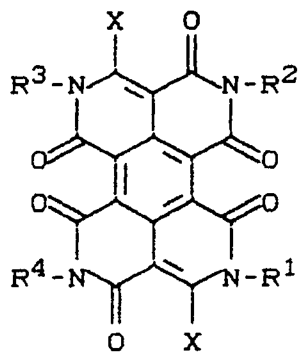

(VIa)

bzw.

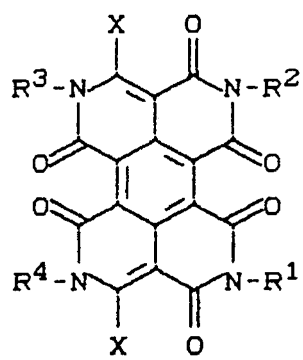

(VIb)

mit $R^1$ bis $R^4$ = Alkyl, Cycloalkyl oder Aralkyl und X = Halogen erhält man aus den entsprechenden Verbindungen mit X = OH durch Umsetzung mit Halogenierungsmitteln, beispielsweise $PCl_5$, $PBr_5$ oder $POCl_3$ bei etwa 120 bis 200 °C, vorzugsweise 130 bis 18 °C, gegebenenfalls in Gegenwart inerter Lösungsmittel wie Nitrobenzol oder Dichlorbenzol. Die Verbindungen VIa bzw. VIb stellen orangefarbene schwerlösliche Festkörper dar.

Verbindungen der Formel

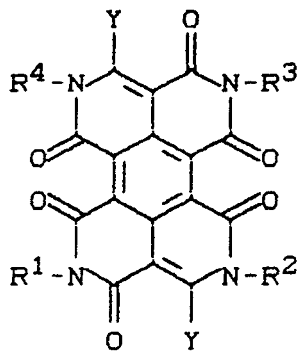

(VIIa)

bzw.

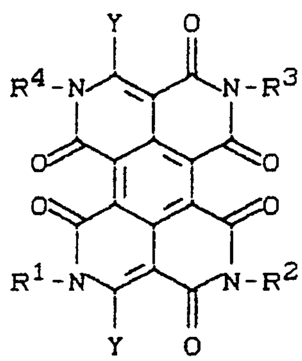

(VIIb)

mit Y = $NR^1R^2$, $SR^1$ oder $OR^1$ erhält man durch Umsetzung von VIa/VIb mit X = Halogen mit Nucleophilen Y-H (VIII) bei Temperaturen von etwa 20 bis 200 °C, vorzugsweise 40 bis 140 °C, gegebenenfalls in einem inerten Lösungsmittel und in Gegenwart säurebindender Mittel wie Alkalicarbonaten, -hydrogencarbonaten oder -acetaten. $R^1$ und $R^2$ haben die oben angegebenen Bedeutungen.

Geeignete Verbindungen VIII sind beispielsweise Ammoniak, gegebenenfalls substituierte aromatische oder aliphatische Amine, Alkohole, Phenole oder Thiophenole oder Mercaptane oder deren Salze. Bei Verwendung von Alkoholaten, Phenolaten oder Thiophenolaten sind bevorzugtes Lösungsmittel die entsprechenden Alkohole.

Verbindungen der Formel

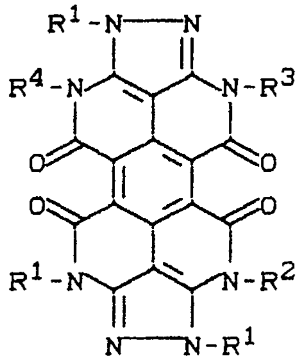

(IXa)

bzw.

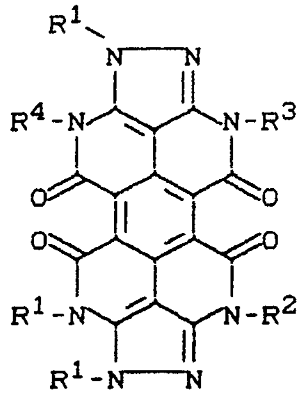

(IXb)

erhält man durch Umsetzung von VIa/VIb (X = Halogen) mit Hydrazinen der Formel

$R_1NH\text{-}NH_2$ (X)

worin $R_1$ die oben angegebene Bedeutung hat, bei ca. 100 bis 200 °C, vorzugsweise 140 bis 180 °C, gegebenenfalls in Gegenwart inerter Lösungsmittel und säurebindender Mittel.

Die Verbindungen IXa/IXb stellen blaue bis grüne Pigmente dar.

Verbindungen der Formel

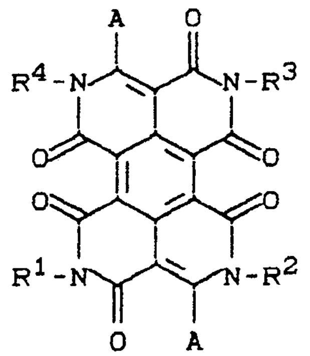

(XIa)

bzw.

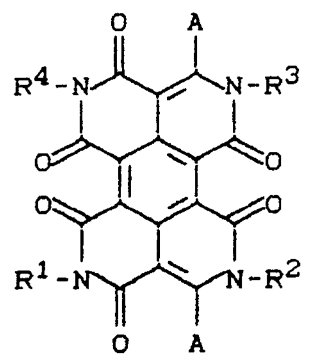

(XIb),

erhält man durch Umsetzung von VIa/VIb mit X = Halogen mit aromatischen Verbindungen der Formel

A - H (XII)

worin A einen gegebenenfalls substituierten Aryl- oder Hetarylrest bedeuten, beispielsweise solche, wie sie für $R^1$ bis $R^4$ beschrieben sind, in Gegenwart von Friedel-Crafts-Katalysatoren.

Im allgemeinen setzt man einen Überschuß von XII und mehr als äquimolare Mengen des Katalysators ein, z.B. $AlCl_3$, und arbeitet bei etwa 100 bis 200 °C, gegebenenfalls in Gegenwart eines inerten Lösungsmittels. Es wird wie üblich aufgearbeitet.

Die Verbindungen XIa/XIb kann man auch aus VIa/VIb (X = Halogen) und metallorganischen Verbindungen, beispielsweise Phenylmagnesiumbromid in inerten Lösungsmitteln herstellen.

In Abhängigkeit von Rest A handelt es sich bei XIa/XIb um Pigmente oder lösliche Farbstoffe.

Beispiel 1

26 g des trockenen Dinatriumsalzes von IV a/b werden in 90 ml Toluolsulfonsäuremethylester 12,5 Stunden lang bei 190 °C Innentemperatur gerührt. Dabei werden nach und nach zur Verbesserung der Rührbarkeit des Reaktionsgemisches 45 ml o-Dichlorbenzol zugegeben.

Nach 12,5 Stunden wird auf 100 °C abgekühlt, dann werden 150 ml Methanol zugegeben und kaltgerührt. Es wird bei Raumtemperatur abgesaugtg das Nutschgut in Wasser angerührt, erneut abgesaugt und getrocknet. Man erhält 23 g einer Verbindung der Formel

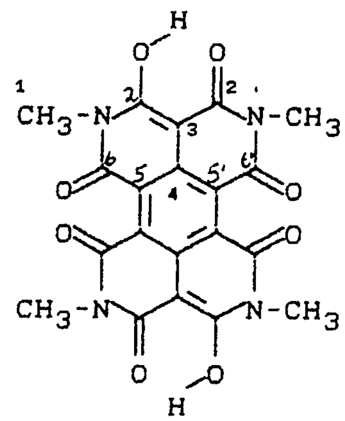

(XIIIa)

bzw.

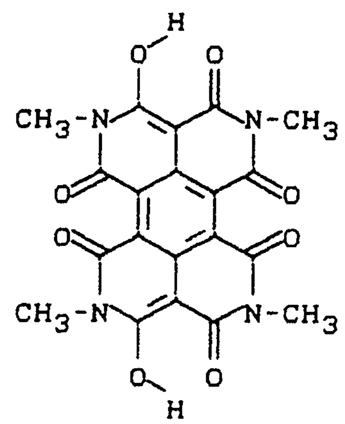

(XIIIb),

als ein rotviolettes Pigment mit sehr guten Überlackierechtheiten und Migrationseigenschaften.
IR-Daten: 2965, 1710, 1695, 1600, 1420, 1375, 1325, 1255, 1180, 1070, 1005, 830, 790 $cm^{-1}$
$^{13}C$-NMR-Daten (Festkörper-CPMAS)
$\delta$ = 28,5 ($N-CH_3$), 82,3 (C-3), 122,6 (C-5, C-5′), 129,4 (C-4), 158,9 (C-6, C-6′), 165,1 ppm (C-2, C-2′).

Bei Verwendung entsprechender Mengen Octyl-, Hexadecyl-oder Menthyltosylat erhält man lösliche Farbstoffe mit denen sich beispielsweise Polystyrol blau einfärben läßt.

Beispiel 2

5 g der Verbindung XIII werden zusammen mit 5,7 g $Ni(OAc)_2 \cdot 4\ H_2O$ in 30 Teilen DMF 80 Minuten lang bei 100 bis 110 °C gerührt. Danach wird abgesaugt und mit Methanol gewaschen. Man erhält 5,9 g eines blauen Pigmentes, welches über ausgezeichnete Überlackier- und Lichtechtheiten verfügt.

Beispiel 3

5 g der Verbindung XIII werden in 100 ml einer ca. 2 %igen, wäßrigen $NH_3$-Lösung 24 Stunden lang bei Raumtemperatur gerührt. Nach Absaugen erhält man 4,4 g eines blauen Pigmentes, welches ähnliche Eigenschaften besitzt wie das in Beispiel 2 beschriebene.

Ausweislich des Festkörper-$^{13}C$-NMR-Spektrums liegt in diesem Salz die Verbindung XIII als Dianion der Formel

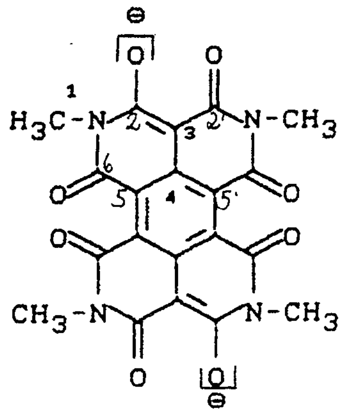

bzw.

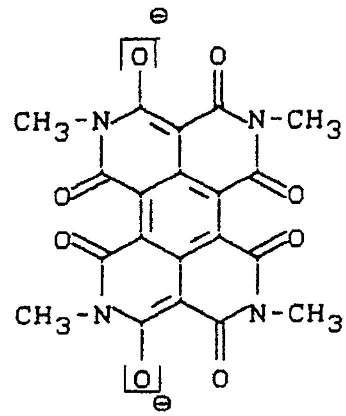

(XIIIb),

vor.

$^{13}$C-NMR-Daten: $\delta$ = 25,1 (C-1), 84,5 (C-3), 115,2, 116,2 (C-5, C-5'), 128,7 (C-4), 159,1, 159,5 (C-2, C-2'), 161,0 ppm (C-6, C-6').

Beispiel 4

11,8 g der Verbindung XIII werden zusammen mit 10,9 g $PCl_5$ in 250 ml Nitrobenzol 5,5 Stunden lang bei 110 bis 120 °C gerührt. Nach dieser Zeit werden weitere 2,0 ml $PCl_5$ nachgesetzt und nochmals 2 Stunden bei obengenannter Temperatur gerührt. Dann wird bei 65 °C abgesaugt und mit Nitrobenzol gewaschen. Der Nutschkuchen wird in 200 ml Methanol angerührt und erneut abgesaugt. Man erhält 11,3 g eines orangefarbenen Feststoffs der Formel

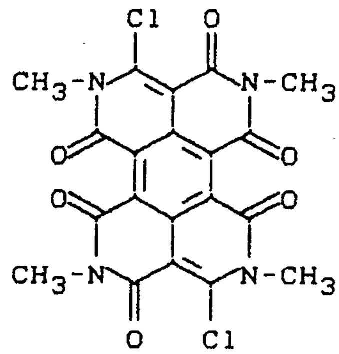

(XIVa)

bzw.

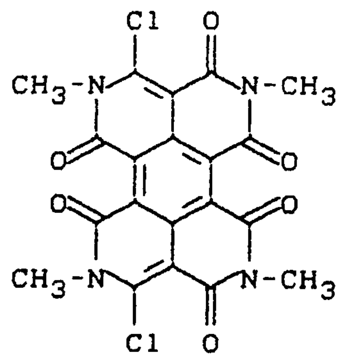

(XIVb),

UV/VIS λ = 490 nm (ε = 5450)
IR: γ = 1715, 1660, 1550, 1510, 1475, 1330, 1290, 1080 $cm^{-1}$

Beispiel 5

20 g XIV werden zusammen mit 13 g 4-Chloranilin und 12 ml Triethylamin in 250 ml Nitrobenzol 5 Stunden lang bei 110 bis 120 °C gerührt.

Nach Abkühlen auf Raumtemperatur und Zugabe von 300 ml Methanol wird abgesaugt und gründlich mit Methanol gewaschen, um das Triethylaminhydrochlorid zu entfernen. Man erhält 22 g der Verbindung der Formel

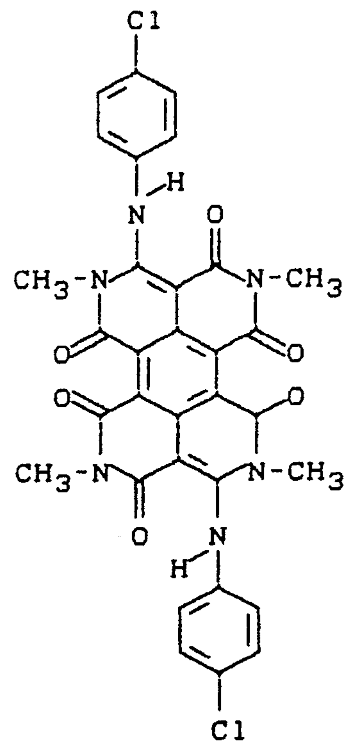

bzw.

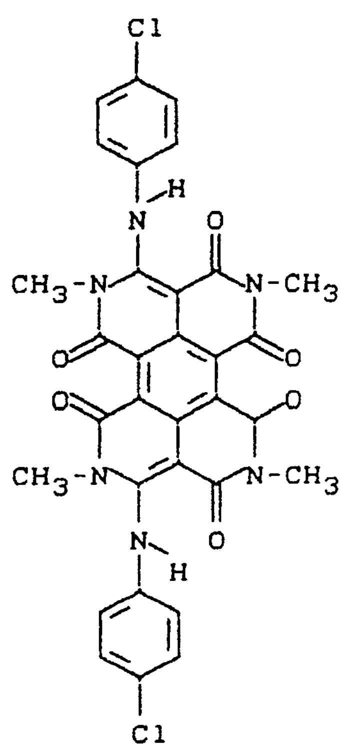

IR-Daten: $\gamma$ = 1700, 1630, 1605, 1580, 1490, 1445, 1120, 1100, 795 $cm^{-1}$
$\lambda_{max}$ = 581 nm

Analog Beispiel 5 lassen sich durch Umsetzung mit den Nucleophilen R-H die Verbindungen der

folgenden Tabelle herstellen:

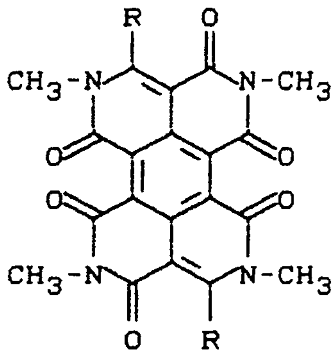

bzw.

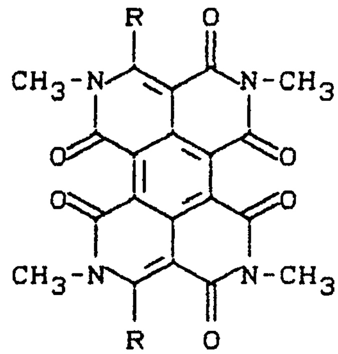

| Bsp. | R | Farbton | λmax [nm] | Bemerkungen |
|---|---|---|---|---|
| 5 | 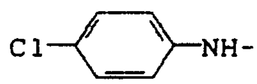 | blau | 581 | löslicher Farbstoff |
| 6 | 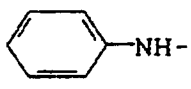 | blau | 586 | löslicher Farbstoff |
| 7 | 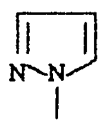 | orange | 478 | löslicher Farbstoff |
| 8 | 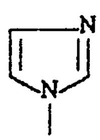 | orange | 483 | löslicher Farbstoff |
| 9 | 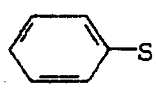 | rot | 524 | löslicher Farbstoff |
| 10 | 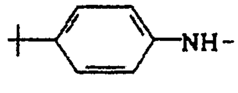 | blau | 588 | löslicher Farbstoff |
| 11 | 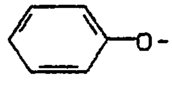 | rot | 480 | löslicher Farbstoff |

Beispiel 12

20 g XIV werden mit 13 g Aluminiumchlorid vermischt und in 90 ml N,N-Diethylanilin angeschlämmt. Es wird auf 180° C aufgeheizt und bei dieser Temperatur 5 Stunden lang gerührt. Danach wird abgekühlt, mit 500 ml Ethanol verrührt, abgesaugt, in 500 ml Wasser angeschlämmt und mit verdünnter Salzsäure leicht angesäuert. Nach Absaugen, Neutralwaschen und Trocknen erhält man 25 g eines orangefarbenen Pigments der Formel

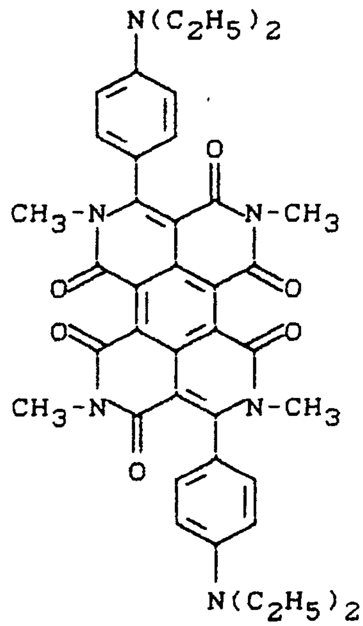

bzw.

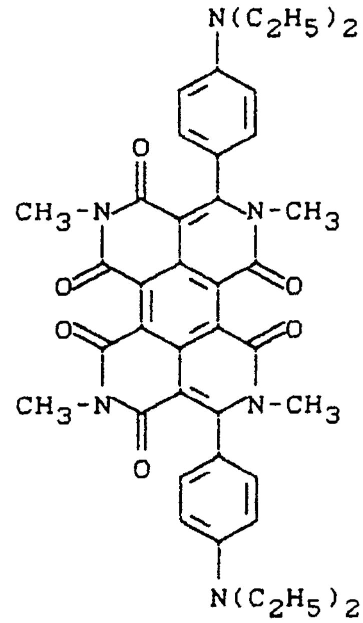

Beispiel 13

0,2 g Pigment nach Beispiel 1 werden mit 100 g Polyethylen-, Polypropylen oder Polystyrolgranulat gemischt. Die Mischung kann entweder bei 220 bis 280° C direkt in einer Spritzgußmaschine verspritzt oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgußmaschine verspritzt.

Die rotvioletten Formlinge besitzen sehr gute Licht- und Migrationsechtheit. In ähnlicher Weise können bei 280 bis 300° C, gegebenenfalls unter Stickstoffatmosphäre, synthetische Polyamide aus Caprolactam oder Adipinsäure und Hexamethylendiamin oder die Kondensate aus Terephthalsäure und Ethylenglykol gefärbt werden.

Beispiel 14

4 g feingemahlenes Pigment gemäß Beispiel 1 werden in 92 g eines Einbrennlackes folgender Zusammensetzung dispergiert:
33 % Alkydharz
15 % Melaminharz
5 % Glykolmonomethylether
34 % Xylol
13 % Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden. Nach erfolgter Dispergierung wird der pigmentierte Lack auf Papier-, Glas-, Kunststoff- oder Metall-Folien aufgetragen und 30 Minuten bei 130° C eingebrannt. Die Lackierungen besitzen sehr gute Licht- und Wetterbeständigkeit sowie gute Überlackierechtheit.

Dieser Einbrennlack wird auf weißes Papier aufgestrichen, bei 130° C eingebrannt und zeigt einen rotvioletten Farbton.

**Ansprüche**

1. Heterocyclische Verbindungen der Formel

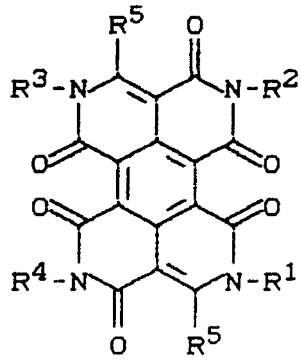

(Ia)

bzw.

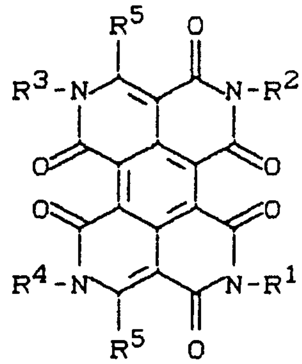

(Ib),

worin bedeuten:
$R^1$, $R^2$, $R^3$, $R^4$ = H, Alkyl, Cycloalkyl, Aralkyl, Aryl oder Hetaryl,
$R^5$ = Halogen, $NR^1R^2$, $SR^1$, $OR^1$, Aryl, Hetaryl oder OKat, worin
Kat = Kation, wobei für $R^1$ bis $R^4$ = H $R^5$ nicht für OH oder ONa steht.

2. Verbindungen des Anspruchs 1 der Formel

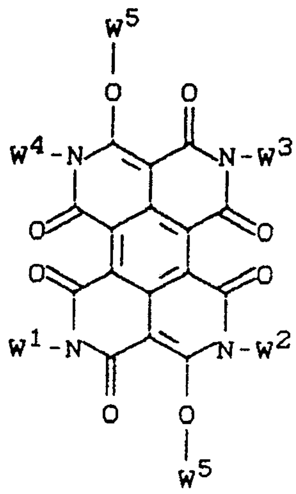

(Ic)

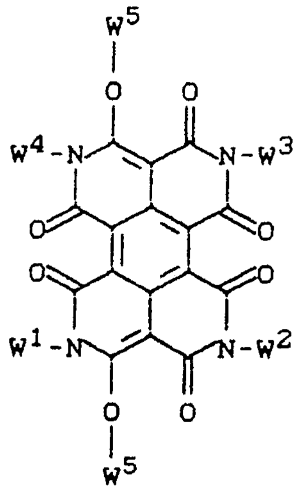

(Id),

worin $W^1$ bis $W^4$ = gegebenenfalls substituiertes Alkyl-bzw. Cycloalkyl und $W^5$ = Wasserstoff oder gegebenenfalls substituiertes Alkyl- oder Cycloalkyl oder Kat. bedeuten.

3. Verbindungen des Anspruchs 2 mit $W^1$ bis $W^4$ = unsubstituiertes $C_1$-$C_{20}$-Alkyl und $W^5$ = Wasserstoff, gegebenenfalls substituiertes Ammoniumion oder Metallkation.

4. Verbindungen des Anspruchs 1 der Formel

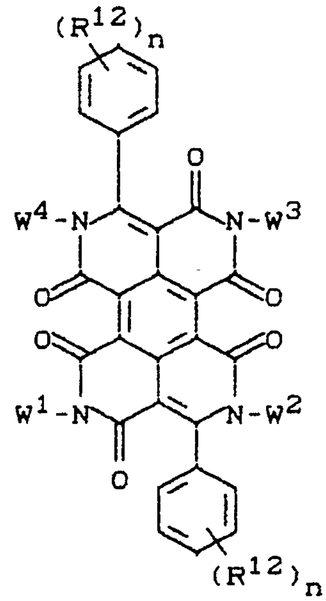

(IIa)

bzw.

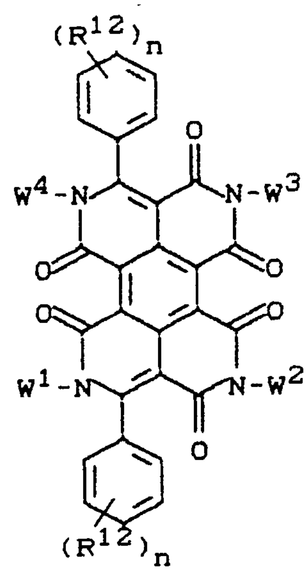

(IIb)

wobei

$W^1$ bis $W^4$ die in Anspruch 3 angegebenen Bedeutungen haben und

$R^{12}$ = Cl, Br, F, CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SOOCOR^{10}$ und $OCONR^8R^9$ mit den obengenannten Substituentenbedeutungen für $R^6$ bis $R^{10}$ und n = 0-3,

wobei

$R_6$ = gegebenenfalls substituiertes Alkyl oder Cycloalkyl,

$R_7$-$R_9$ = gegebenenfalls substituiertes Alkyl, Cycloalkyl, Aralkyl, Aryl oder heterocyclischer Rest, wobei $R_8$ und $R_9$ zusammen mit dem N-Atom einen 5- oder 6-gliedrigen heterocyclischen Ring bilden können und

$R^{10}$ = H, gegebenenfalls substituiertes Alkyl, Cycloalkyl, Aralkyl oder Aryl bedeuten.

5. Verbindungen der Formel

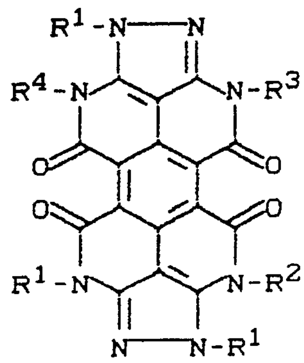

(IXa)

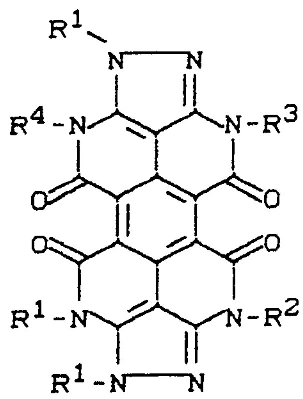

(IXb)

worin $R_1$-$R_4$ die in Anspruch 1 angegebene Bedeutung haben.

6. Verwendung der Verbindungen der Ansprüche 1 - 5 zum Färben oder Pigmentieren von hochmolekularem organischen Material oder als Lackpigmente.